# EUROPEAN PATENT APPLICATION

(11) **EP 4 372 074 A1**
(43) Date of publication of application: **22.05.2024**
(21) Application number: 22208565.6
(22) Date of filing: 21.11.2022
(51) Int. Cl.: C12M 3/00, C12M 3/06, C12M 1/26, C12M 1/42, C12M 1/34

(54) **METHOD FOR IN VITRO CULTURE UNDER FLOW AND FLOW CHAMBER THEREFOR**

(71) Applicant: Medizinische Universität Graz, 8036 Graz (AT)
(72) Inventor: BRUGGER, Beatrice Anna, 8010 Graz (AT); PICHLER, Markus, 8182 Puch bei Weiz (AT); GAUSTER, Martin, 8075 Hart bei Graz (AT)
(74) Representative: SONN Patentanwälte GmbH & Co KG

(57) **Abstract**

Disclosed is a method for *in vitro* culture under laminar flow, comprising the steps of: providing a flow chamber, loading a tissue sample onto a first loading position in the flow chamber, culturing the tissue sample under a laminar cell culture medium flow in the flow chamber, wherein a part of the tissue sample becomes necrotic, and harvesting at least a portion of the tissue sample after the culturing by separating the portion from necrotic tissue. Also disclosed is a flow chamber for *in vitro* culture under laminar flow according to this method. The flow chamber comprises a first loading position for a tissue sample and preferably a second loading position for a cell sample.

## Description

The present invention relates to methods for *in vitro* culture under laminar flow, in particular under low or high shear stress (or under physiological or pathological shear stress), and flow chambers for this purpose.

In many standard *in vitro* experiments, cells are cultured without flow, which does not correspond to physiological conditions. Without flow, shear stress-dependent cellular changes cannot be observed. In contrast, *in vitro* cell culture under laminar flow, in particular unidirectional laminar flow, induces a more physiological, in vivo-like behaviour in cells.

Accordingly, methods for culturing cells under laminar flow, in particular under low or high shear stress, and flow chambers fit for this purpose have been provided in the prior art.

Such methods and flow chambers are known e.g. from US 11,033,897 B2. This document discloses a microfluidic device including a chamber having a fluid inlet, a fluid outlet and a sealable port. The chamber may be sized so that it can contain a number of cells whilst still allowing fluid to flow through the chamber from the fluid inlet to the fluid outlet in a laminar flow fashion.

Along similar lines, WO 2005/123258 A1 relates to a bioreactor for studying the effects of imposed stimuli on cellular activity. The culture chamber of the bioreactor may be made of silicone rubber and be shaped in such a way that through said culture chamber a laminar flow of the culture medium is achieved.

US 10,421,936 B2 relates to a system and method for microfluidic cell culture. Microfluidic cell culture arrays disclosed therein may exploit laminar flow to deliver the cells to the chambers and then to ensure that the cells are not disturbed by subsequent perfusion.

Duan et al. (Duan, Sujuan, et al., Journal of ophthalmology 2021) studied the response of corneal endothelial cells to shear stress in an *in vitro* flow model. As disclosed therein, a parallel plate flow chamber (Glycotech, Gaithersburg, MD, USA) was used to expose cell monolayers to laminar shear stress.

Flow chambers and systems for subjecting cell cultures to laminar flow are also available commercially, e.g. from ibidi GmbH, Germany.

However, the methods and flow chambers known in the prior art do not sufficiently address the need of culturing tissue samples under laminar flow.

Accordingly, it is an object of the present invention to provide methods and flow chambers for culturing tissue samples under laminar flow, in particular under low or high shear stress (or under physiological or pathological shear stress).

The present invention provides a method for *in vitro* culture under laminar flow, comprising the steps of:
- providing a flow chamber,
- loading a tissue sample, preferably a placental tissue sample (in particular a villous tissue sample such as villous explants) or a lung tissue sample, onto a first loading position in the flow chamber,
- culturing the tissue sample under a laminar cell culture medium flow (in particular unidirectional laminar flow) in the flow chamber, wherein a part of the tissue sample becomes necrotic, and
- harvesting at least a portion of the tissue sample after the culturing by separating the portion from necrotic tissue.

By way of example, a "laminar cell culture medium flow" is established by pumping cell culture medium (which preferably has a temperature of 37°C) through the cell chamber in a manner such that a (unidirectional) laminar flow is maintained.

The above-mentioned harvesting step may then be followed e.g. by downstream analysis such as gene expression profiling and/or a number of molecular investigations (protein analysis, metabolic profiling), or microscopy, or further culturing in another flow cell, e.g. to bring the portion of the tissue sample into contact with a test compound under laminar flow (e.g. to examine whether this test compound is able to enter the cells under these flow conditions).

Typically, the tissue sample comprises at least three different cell types (such as epithelial cells, endothelial cells and immune cells) and an extracellular matrix. It may also comprise blood vessels. It is preferred that the tissue sample has been (directly) obtained by biopsy from an individual.

The tissue sample used in the present invention preferably has a minimum diameter (i.e. the result obtained when measuring its shortest extension) of at least 0.5 mm, preferably 1.0 mm, more preferably at least 1.5 mm, even more preferably at least 2.0 mm, yet even more preferably at least 2.5 mm, especially at least 3.0 mm (when it is loaded into the flow chamber). Alternatively, or in addition thereto, the tissue sample has a plurality of cell layers (extending into three dimensions), preferably at least three cell layers, more preferably at least four cell layers, especially at least five cell layers. In particular, the tissue sample may contain cell layers of different types. Preferably, during the culturing, at least a portion (e.g. at least 50% or at least 60% or at least 70% or at least 80%) of the cell layers of the tissue sample are maintained.

The present invention also provides a flow chamber for *in vitro* culture under laminar flow (according to the above method). The flow chamber comprises a first loading position for a tissue sample and preferably a second loading position for a cell sample.

On particular problem when culturing tissue samples (especially placental tissue samples) under laminar flow is that necrotic tissue will typically form in areas used to attach the tissue sample to the flow chamber such that it is not swept away by flow. When this necrotic tissue is harvested and included into the downstream analysis (e.g. mRNA or protein expression profiling), error was introduced into the analysis, as found in the course of the present invention. The present invention addresses this problem by foreseeing an appropriate separation step during the harvesting.

The present invention is particularly useful for the ex *vivo* study of placental tissue, which is generally subjected to a shear stress in the range of 0.01-12 dyn/cm2 during pregnancy.

When a second loading position for a cell sample is provided, it is also useful for correlative approaches between cells and tissues from the same organ (in particular placental cell samples and placental tissues), with the aim that the cells and tissues are subjected to the same laminar flow conditions (and the same shear stress) during the culturing.

Unrelated to subjecting cells or tissues to laminar flow, WO 2021/183527 A1 discloses systems, method, and devices for ex vivo analysis of resected tissue samples.

*Ex vivo* studies involving placenta tissues or cells are for instance disclosed in Kupper et al. (Kupper, Nadja, et al. "Placental villous explant culture 2.0: flow culture allows studies closer to the in vivo situation." International journal of molecular sciences 22.14 (2021): 7464.) and Lee et al. (Lee, Ji Soo, et al. "Placenta-on-a-chip: a novel platform to study the biology of the human placenta." The Journal of Maternal-Fetal & Neonatal Medicine 29.7 (2016): 1046-1054.). However, these documents are entirely silent on achieving laminar flow.

There are many cell types that are constantly subjected to fluid shear stress in the human or animal body. For instance, endothelial cells from vascular blood vessels and lymphatic system are exposed to circulating blood and lymph. Depending on the vessel type, shear stress may vary from e.g. 30 dyn/cm² in arteries to 1 dyn/cm² in venous vessels and capillaries. It was reported that endothelial shear stress alters cytoskeletal organization, cell shape and gene expression, increasing intracellular calcium concentration, triggering nitric oxide production and producing changes in actin stress fiber formation, aligning and remodelling microfilament network in the direction of flow.

Epithelial cells from various tissues are also exposed to shear stress forces in the human or animal body. The kidney, in its filtering function through a sophisticated tubular network, is capable of filtrating 120 ml per minute, which means 180 L per day. Accordingly, the epithelial cells lining every nephron are exposed to the shear stress of glomerular filtrate, estimated between 0.1-1 dyn/cm². Some studies demonstrated that renal proximal tubule epithelial cells lead to cilia formation in the presence of shear stress. In addition, the mediated signal transduction is regulated, improving epithelial cytoarchitecture, leading to increased cell volume, greater polarization of aquaporins, cation transporters and ion channels than kidney epithelial cells in static culture.

In the body, shear stress is also generated on the air side of the alveolar-capillary barrier. At every breath taken, epithelial cells lining the airways are exposed to shear stress generated by the air flow, which at rest breathing is 0.5-3 dyn/cm². These forces have been demonstrated to modulate airway epithelial barrier function, mucous production and ciliary beating alignment.

Also, in the human intestinal tract the flow of digesta induced by the peristaltic motion of the intestinal wall affects the enterocytes lining the digestive tube, which has been previously shown to be 0.002-0.08 dyn/cm².

As mentioned, the human placenta is also exposed to shear stress, depending on the state of pregnancy and if it is a healthy pregnancy or not, it can e.g. vary from 0.01-5 dyn/cm2

Depending on the research question and the cell line or tissue cultured under flow, the shear rate can be adapted to physiological or pathological conditions.

The methods and flow chambers of the present invention can be used for a variety of studies. Not only observing which effect flow itself has on the cells or tissues, it can be also useful to observe if test compounds can enter the cells under different flow rates or if nanoparticles are taken up at different flow rates or in specific cells treated under flow conditions.

According to a preferred embodiment, the necrotic tissue remains in the first loading position in the flow chamber after the harvesting. The flow chamber may then be cleaned (e.g. by cleaning with ethanol and heat sterilization) for re-use with another cell sample or tissue sample.

In an especially preferred embodiment, the first loading position is at a recess for clamping the tissue sample in the flow chamber, wherein the loading step comprises clamping the tissue sample at the recess. During the culturing, the tissue sample may remain clamped at the recess (or groove). Typically, the part of the tissue sample which remains in the recess during the culturing becomes necrotic. By way of example, the portion of the tissue sample to be harvested may be easily separated from this necrotic (and undesired) part by cutting with scissors.

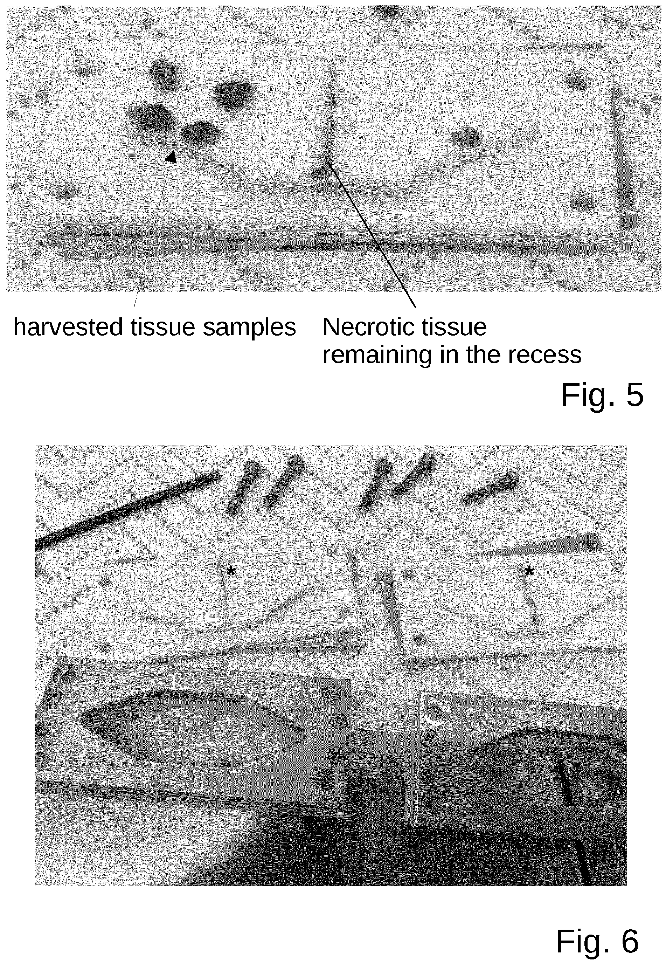

In embodiments, the flow chamber may contain several loading positions of the type of the first loading position (e.g. to load several tissue samples). These loading positions may for instance be located all at the same recess in the flow chamber if present (cf. e.g. Fig. 5) or, when the flow chamber comprises several recesses, at different recesses in the flow chamber.

According to another preferred embodiment, the main axis of the recess is preferably oriented at an angle of at least 10° to the laminar flow direction, preferably at least 30°, more preferably at least 50°, even more preferably at least 60°, yet even more preferably at least 70°, especially at least 80°. In particular, the main axis of the recess is essentially orientated perpendicularly to the direction of the laminar flow. This may decrease the likelihood of the tissue sample being swept away by the flow, while also decreasing the risk of inducing turbulence.

In yet another preferment, the shear stress acting upon a portion of the tissue sample in the first loading position is at least 0.25 dyn/cm², preferably 0.5 dyn/cm², more preferably at least 1.0 dyn/cm², yet more preferably at least 2.5 dyn/cm², even more preferably at least 5.0 dyn/cm², yet even more preferably at least 7.5 dyn/cm², especially at least 10.0 dyn/cm² or even at least 10.0 dyn/cm² at 37°C for at least 1 hour during the culturing. Preferably, this shear stress lasts for at least 2 hours, more preferably for at least 4 hours preferably for at least 8 hours, yet more preferably for at least 12 hours, even more preferably for at least 24 hours, yet even more preferably for at least 48 hours, especially for at least 72 hours. In embodiments, the shear stress may last for at least 1 day, more preferably for at least 2 days, even more preferably for at least 4 days, yet more preferably for at least 6 days, even more preferably for at least 8 days, yet even more preferably for at least 12 days, especially for at least 24 days.

Upon having read the present specification, the skilled person is able to choose or modify the dimensions of the flow chamber, for instance to accommodate more and/or larger samples.

To enable correlative approaches between cells and tissues (in particular placental cell samples and placental tissues), with the aim that the cells and tissues are subjected to the same laminar flow conditions (and the same shear stress) during the culturing, the method comprises, in another preferred embodiment, the following steps:
- providing a flow chamber,
- loading a cell sample onto a second loading position in the flow chamber,
- culturing the cell sample under a laminar cell culture medium flow in the flow chamber, and
- harvesting at least a portion of the cell sample after the culturing.

The flow chamber provided may be the same as used before for the tissue sample, after cleaning. The recess of the flow chamber (if present) may be covered with a removeable plate (e.g. a glass plate) to provide the second loading position.

In embodiments, the flow chamber may contain several loading positions of the type of the second loading position (e.g. to load several cell samples). These loading positions may for instance be located all on the same glass plate.

In embodiments, the cell sample may be selected from any primary cell line or any tumor cell line. The cell sample may comprise several cell types. The cells of the cell sample may e.g. be endothelial cells or epithelial cells.

Typically, the cell sample is in the form of a monolayer (or di-layer or tri-layer).

According to a preferred embodiment, the cell sample comprises cytotrophoblast and syncytialized syncytiotrophoblast cells.

In yet another preferred embodiment, essentially the same shear stress acts upon at least a portion of the cell sample in the second loading position and at least a portion of the tissue sample in the first loading position during the culturing for at least 1 hour, preferably for at least 2 hours, more preferably for at least 4 hours preferably for at least 8 hours, yet more preferably for at least 12 hours, even more preferably for at least 24 hours, yet even more preferably for at least 48 hours, especially for at least 72 hours. (In embodiments, essentially the same shear stress may act upon at least a portion of the cell sample in the second loading position and/or at least a portion of the tissue sample in the first loading position during the culturing for at least 1 day, more preferably for at least 2 days, even more preferably for at least 4 days, yet more preferably for at least 6 days, even more preferably for at least 8 days, yet even more preferably for at least 12 days, especially for at least 24 days.) By way of example, these correlative experiments may be run in parallel (i.e. with multiple flow chambers, whereby the shear stress is preferably applied at the same time to both samples) or in sequence (optionally even with the same flow chamber, after cleaning).In another preferred embodiment, the cell sample and the tissue sample contain at least one cell of the same type.

According to a further preference, the cell sample and the tissue sample each contain at least one (human) placental cell. Alternatively, or in addition thereto, the (human) cell sample and the (human) tissue sample may preferably each contain at least one: ovarian cell (such as cilia cells, or granulosa cells), umbilical vein endothelial cell, pulmonary epithelial barrier cell, human stem cell, smooth muscle cell, skin cell (e.g. epidermal cells, skin fibroblasts), epithelial cell (such as kidney tubular epithelial cells or urothel epithelial cells) or endothelial cell. The tissue sample in particular may contain mixtures of any one of these cells.

According to another preferred embodiment, the tissue sample is a placental tissue sample or a lung tissue sample.

Alternatively, or in addition thereto, the cell sample and/or the tissue sample may be human.

In yet another preferred embodiment, the method comprises analysing the harvested tissue sample portion and the harvested cell sample portion.

According to another preferred embodiment, the first loading position is at a recess for clamping the tissue sample in the flow chamber.

Another preference relates to the second loading position being at a removable plate (such as a removable glass plate) which covers the recess. Accordingly, the flow chamber may comprise the removable plate.

As used herein, the "A" and "B" being "essentially" the same (such as the shear stress being essentially the same) refers to A having the same value as B or A being within a -25% to +25% range, preferably a -10% to +10% range, of B (wherein the percentage is calculated from B).

The present invention is further described by the following examples and the figures, yet without being restricted thereto.

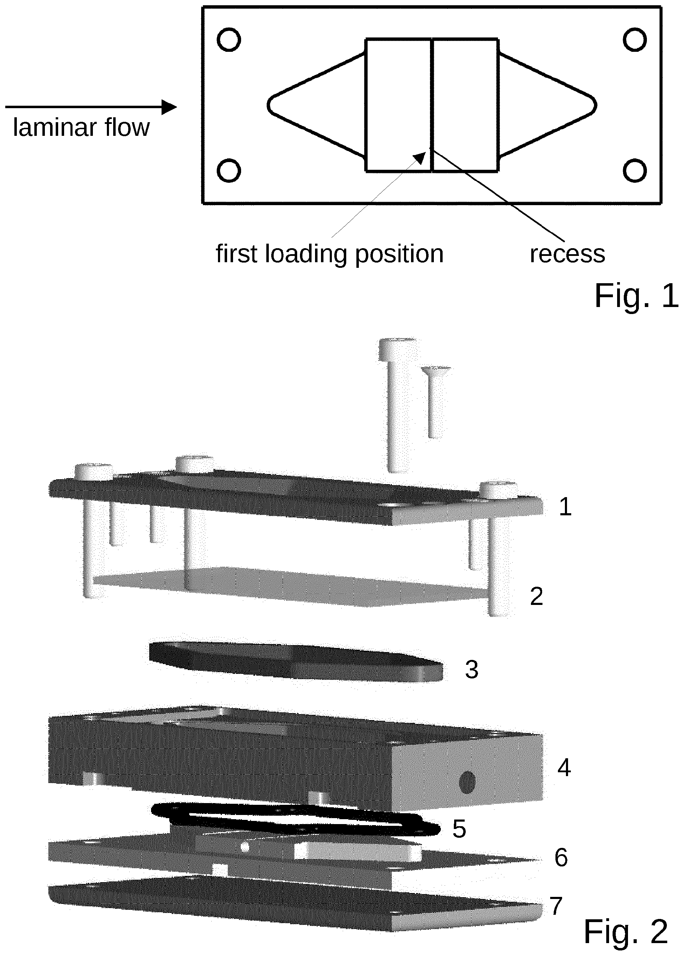

Fig. 1 - Top view of the sample carrier plate of the flow chamber with the recess of the first loading position shown. The axis of the recess is oriented perpendicularly to the direction of the laminar flow.
Fig. 2 - Exploded view drawing of the flow chamber. 1: anodized aluminium top, 2: glass cover, 3: silicon seal, 4: main housing (e.g. made of PTFE), 5: rubber seal, 6: sample carrier plate (e.g. made of PTFE), 7: anodized aluminium bottom.

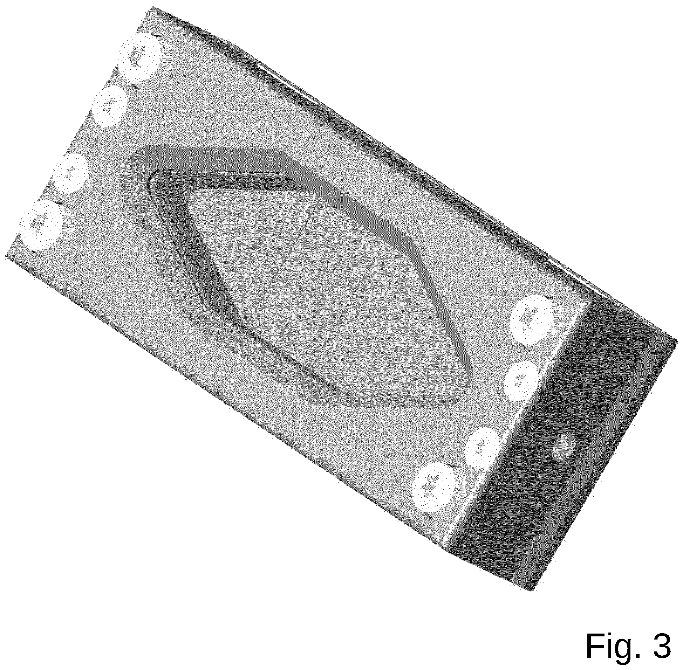

Fig. 3 - 3D view of the flow chamber.

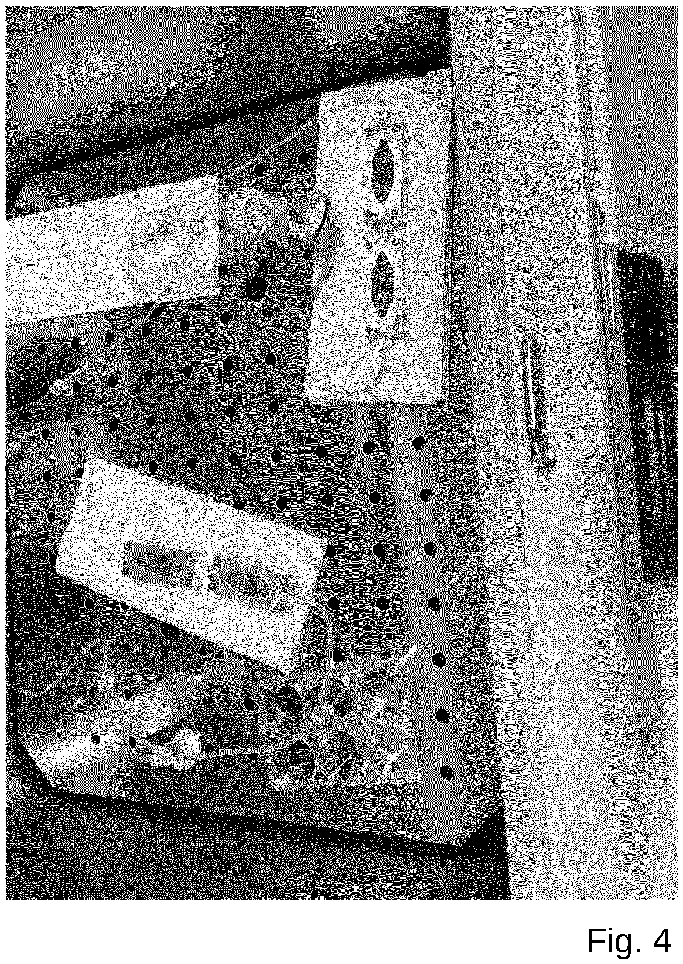

Fig. 4 - Photograph of four flow chambers in use.
Fig. 5 - Photograph of harvested tissue sample portions separated from their respective necrotic tissue which remains in the recess of the flow chamber after harvesting.
Fig. 6 - Photograph of necrotic tissue (*) remaining in the respective recess of the flow chambers after harvesting.

### Example 1 - Tissue culture under laminar flow

For tissue culturing under laminar flow conditions, a placental tissue was collected freshly after caesarean section from the clinic. Within 1-4h the tissue was cut in 6x6 mm pieces, yielding several tissue samples for culturing. After washing in PBS, each tissue sample is loaded into the respective first loading position of a flow chamber. Typically, this was done by picking the stem of a villi (or another small piece of the tissue) with a forceps and clamping it into the recess.

Cell culture medium was placed in the reservoir bottle. The flow circuit was subjected to a pump system, to start the culturing under laminar flow of the cell culture medium (e.g. at 1.0 dyn/cm² for 2 hours). The duration of the experiment depended on the research question and was performed from one hour to several days.

After the culturing under laminar flow, the flow chambers were opened, the tissue samples were cut with scissors above the recess. Thereby, parts of the tissue samples which became necrotic (and would introduce error into downstream analysis) remained in the recess of the respective flow chamber and only the non-necrotic portions of the tissue samples was harvested.

These portions of the tissue samples were then subjected to the downstream analyses relevant for the research question. For instance, immunofluorescence, immunohistology, protein determination, western blot, qPCR, mass spectrometry, and/or *nuclear magnetic resonance* may be performed as downstream analysis.

### Example 2 - Correlative cell culture under laminar flow

One of the flow chambers used in Example 1 was cleaned with ethanol and autoclaved.

For a correlative cell culture experiment, BeWo cells were seeded onto a 2x2 mm glass plate. After incubation and attachment of the cell sample to the glass plate, the glass plate was inserted into the flow chamber at the second loading position to cover the recess.

The cell sample was then cultured under laminar flow as disclosed in Example 1 for tissue cultures, such that essentially the same shear stress acted upon the cell sample.

The cell sample was then harvested for correlative downstream analysis. For instance, immunofluorescence, immunohistology, protein determination, western blot, qPCR and/or mass spectrometry may be performed as correlative downstream analysis.

## Claims

1. A method for *in vitro* culture under laminar flow, comprising the steps of:
- providing a flow chamber,
- loading a tissue sample onto a first loading position in the flow chamber,
- culturing the tissue sample under a laminar cell culture medium flow in the flow chamber, wherein a part of the tissue sample becomes necrotic, and
- harvesting at least a portion of the tissue sample after the culturing by separating the portion from necrotic tissue.

2. The method of claim 1, wherein necrotic tissue remains in the first loading position in the flow chamber after the harvesting.

3. The method of claim 1 or 2, wherein the first loading position is at a recess for clamping the tissue sample in the flow chamber, wherein the loading step comprises clamping the tissue sample at the recess.

4. The method of any one of claims 1 to 3, wherein the shear stress acting upon a portion of the tissue sample in the first loading position is at least 0.25 dyn/cm², preferably 0.5 dyn/cm², more preferably at least 1.0 dyn/cm², yet more preferably at least 2.5 dyn/cm², even more preferably at least 5.0 dyn/cm², yet even more preferably at least 7.5 dyn/cm², especially at least 10.0 dyn/cm² or even at least 10.0 dyn/cm² at 37°C for at least 1 hour during the culturing, preferably for at least 2 hours, more preferably for at least 4 hours preferably for at least 8 hours, yet more preferably for at least 12 hours, even more preferably for at least 24 hours, yet even more preferably for at least 48 hours, especially for at least 72 hours.

5. The method of any one of claims 1 to 4, further comprising the following steps:
- providing a flow chamber,
- loading a cell sample onto a second loading position in the flow chamber,
- culturing the cell sample under a laminar cell culture medium flow in the flow chamber, and
- harvesting at least a portion of the cell sample after the culturing.

6. The method of claim 5, wherein essentially the same shear stress acts upon at least a portion of the cell sample in the second loading position and at least a portion of the tissue sample in the first loading position during the culturing for at least 1 hour, preferably for at least 1 day, more preferably for at least 2 days, even more preferably for at least 4 days, yet more preferably for at least 6 days, even more preferably for at least 8 days, yet even more preferably for at least 12 days, especially for at least 24 days.

7. The method of claim 5 or 6, wherein the shear stress acting upon the portion of the cell sample in the second loading position and the portion of the tissue sample in the first loading position is at least 0.25 dyn/cm², preferably 0.5 dyn/cm², more preferably at least 1.0 dyn/cm², yet more preferably at least 2.5 dyn/cm², even more preferably at least 5.0 dyn/cm², yet even more preferably at least 7.5 dyn/cm², especially at least 10.0 dyn/cm² or even at least 10.0 dyn/cm² at 37°C for at least 1 hour during the culturing, preferably for at least 2 hours, more preferably for at least 4 hours preferably for at least 8 hours, yet more preferably for at least 12 hours, even more preferably for at least 24 hours, yet even more preferably for at least 48 hours, especially for at least 72 hours.

8. The method of any one of claims 5 to 7, wherein the cell sample and the tissue sample contain at least one cell of the same type.

9. The method of claim 8, wherein the cell sample and the tissue sample each contain at least one placental cell, in particular a BeWo cell or a trophoblast cell such as a primary trophoblast cell.

10. The method of any one of claims 5 to 9, comprising analysing the harvested tissue sample portion and the harvested cell sample portion.

11. The method of any one of claims 1 to 10, wherein the laminar flow is a unidirectional laminar flow.

12. The method of any one of claims 3 to 11, wherein the main axis of the recess is essentially orientated perpendicularly to the direction of the laminar flow.

13. A flow chamber for *in vitro* culture under laminar flow according to the method of any one of the previous claims, wherein the flow chamber comprises a first loading position for a tissue sample and preferably a second loading position for a cell sample.

14. The flow chamber of claim 13, wherein the first loading position is at a recess for clamping the tissue sample in the flow chamber.

15. The flow chamber of claim 14, wherein the second loading position is at a removable plate which covers the recess.
